(19) Europäisches Patentamt
European Patent Office
Office européen des brevets

(11) EP 2 624 156 A2

(12) **EUROPEAN PATENT APPLICATION**

(43) Date of publication:
**07.08.2013 Bulletin 2013/32**

(51) Int Cl.:
**G06F 19/00** (2011.01)

(21) Application number: **13151500.9**

(22) Date of filing: **16.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.01.2012 KR 20120009761**

(71) Applicant: **Samsung Electronics Co., Ltd Gyeonggi-do 443-742 (KR)**

(72) Inventors:
• **Yoon, Soo-yeoun**
  **Seoul (KR)**
• **Cho, Bong-hyun**
  **Gyeonggi-do (KR)**
• **Choi, Jun-sik**
  **Gyeonggi-do (KR)**

(74) Representative: **Taor, Simon Edward William Venner Shipley LLP 200 Aldersgate London EC1A 4HD (GB)**

(54) **Method of managing information related to an exercise amount and display apparatus using the same, and server of the display apparatus**

(57)     A method of managing information related to an exercise amount and a display apparatus using the same, and a server connected with the display apparatus are provided. The method measures exercise amount information of at least one user using a plurality of exercise content, transmits the measured exercise amount information to a server, receives exercise amount information integrally calculated from the server, and displays the received exercise amount information on the display apparatus. Therefore, it is possible for the user to intuitively monitor change in his/her health state and maintain his/her health using the plurality of content.

EP 2 624 156 A2

**Description**

Apparatuses and methods consistent with exemplary embodiments relate to a method of managing information for an exercise amount and a display apparatus using the same, and also relate to a server. More particularly, apparatuses and methods consistent with exemplary embodiments relate to a method of managing information for an exercise amount, which helps in maintaining a user's health using a display apparatus and a server.

With the population growing older and more obese, the concern for health care is becoming amplified. In recent years, programs which relate to health care of a user using a personal terminal have increased. For example, health care programs which periodically manage health information and exercise information of a user have been developed.

However, the health care programs merely measure an exercise amount of the user (for example, an exercise time, exercise distance, burned calories, and the like) and store or display the measured exercise amount. Thus, it is difficult for a user to know how to manage his/her body. In addition, the user should attempt to manage their health care using exercise content and display apparatuses.

Therefore, there is a need for technology for managing health information of a user more intuitively and integrally.

One or more exemplary embodiments may overcome the above disadvantages and other disadvantages not described above. However, it is understood that one or more exemplary embodiments are not required to overcome the disadvantages described above, and may not overcome any of the problems described above.

One or more exemplary embodiments is directed to a method of managing information for an exercise amount in which a user integrally manages information for an exercise amount using a plurality of content, at least one display apparatus using the same, and a server of the display apparatus.

According to an aspect of an exemplary embodiment, there is provided a method of managing information related to an exercise amount in a display apparatus. The method may include: measuring exercise amount information of at least one user using a plurality of exercise content and transmitting the measured exercise amount information to a server; and receiving exercise amount information which has been calculated from the server, and displaying the received exercise amount information on the display apparatus.

The exercise amount information may include at least one from among exercise content information used by the user, exercise time information of the user, and information related to burned calories calculated using at least one from among a camera and an arm band.

The method may further include acquiring body information of the user and exercise goal information of the user and transmitting the acquired information to the server.

The body information of the user may include at least one from among a height and a weight of the user. The height of the user may be acquired using a camera and the weight of the user may be acquired by a scale. The exercise goal information of the user may include at least one from among a goal weight of the user, an amount of calories to be burned, an exercise duration, and exercise content to be used by the user. The camera may include a depth camera and the scale may include a digital scale.

The method may further include receiving the body information of the user and the exercise goal information of the user from the server. The displaying may include generating a health care user interface (UI) using at least one from among the body information of the user, the exercise goal information of the user, and the exercise amount information of the user and displaying the generated health care UI.

The health care UI may display the exercise amount information measured using the plurality of content, using a graph related to at least one from among dates and content.

The health care UI may display changed body information of the user and information related to whether the user achieves an exercise goal.

The method may further include displaying an exercise goal input user interface (UI) configured to input the exercise goal information of the user. The exercise goal input UI may include a mission list configured to provide an amount of calories to be burned when a specific mission is performed.

The method may further include analyzing the body information of the user and the exercise amount information of the user and providing recommended exercise content for the user.

According to another aspect of an exemplary embodiment, there is provided a display apparatus. The display apparatus may include: an exercise amount measuring unit configured to measure exercise amount information of at least one user using a plurality of exercise content; a communication unit configured to transmit the measured exercise amount information to a server; and a display unit configured to receive exercise amount information which has been calculated, from the server and display the received exercise amount information.

The exercise amount information may include at least one from among exercise content information used by the user, exercise time information of the user, and information related to burned calories calculated using at least one from among a camera and an arm band. The camera may include a depth camera.

The display apparatus may further include an information acquiring unit configured to acquire body information of the user and exercise goal information of the user. The communication unit may transmit the acquired body information of the user and the acquired exercise goal information of the user to the server.

The body information of the user may include at least one from among a height and a weight of the user. The height of the user may be acquired using a camera and the weight of the user may be acquired by a scale. The exercise goal information of the user may include at least one selected from among a goal weight of the user, an amount of calories to be burned, an exercise duration, and exercise content to be used by the user. The camera may include a depth camera and the scale may include a digital scale.

The display apparatus may further include a user interface (UI) unit configured to generate a health care UI using the body information of the user, the exercise goal information of the user, and the exercise amount information of the user received through the communication unit. The display unit may display the generated health care UI.

The health care UI may display the exercise amount information measured using the plurality of content, using a graph related to at least one from among dates and content.

The health care UI may display changed body information of the user and information related to whether the user achieves an exercise goal.

The display apparatus may further include a UI generating unit configured to generate an exercise goal input UI configured to input the exercise goal information of the user. The exercise goal input UI may include a mission list for providing an amount of calories to be burned when a specific mission is performed.

The display apparatus may further include a control unit configured to analyze the body information of the user and the exercise amount information of the user, and provide recommended exercise content for the user.

According to another aspect of an exemplary embodiment, there is provided a method of managing information related to the exercise amount, of a server which is connected with at least one display apparatus, to manage information related to the exercise amount of a user. The method may include: receiving exercise amount information from the at least one display apparatus and storing the received exercise amount information; calculating the exercise amount information received from the at least one display apparatus to generate integrated exercise amount information; and, transmitting the generated integrated exercise amount information to the at least one display apparatus which transmits the request signal if a request signal is received from the at least one display apparatus.

The method may further include receiving body information of the user and exercise goal information of the user from the at least one display apparatus; and providing recommended exercise content using at least one from among the received body information, the exercise goal information, and the exercise amount information of the user.

According to another aspect of an exemplary embodiment, there is provided a server which is connected with at least one display apparatus to manage information related to an exercise amount of a user. The server may include: a communication unit configured to receive exercise amount information of a user from the at least one display apparatus; a storage unit configured to store the exercise amount information of the user received from the communication unit; and a control unit configured to calculate the received exercise amount information and generate integrated exercise amount information to be transmitted to the at least one display apparatus.

The communication unit may receive body information of the user and exercise goal information of the user from the at least one display apparatus and the control unit may provide recommended exercise content using at least one from among the received body information, the exercise goal information, and the exercise amount information of the user.

Additional aspects of the exemplary embodiments will be set forth in the detailed description, will be obvious from the detailed description, or may be learned by practicing the exemplary embodiments.

The above and/or other aspects will be more apparent by describing in detail exemplary embodiments, with reference to the accompanying drawings, in which:

FIG. 1 is a view illustrating an exercise amount information management system including a display apparatus and a server according to an exemplary embodiment;
FIG. 2 is a block diagram illustrating a configuration of a display apparatus according to an exemplary embodiment.
FIGS. 3(A-C) to 7 are views illustrating UIs provided for health care of a user in a display apparatus according to various exemplary embodiments.
FIG. 8 is a block diagram illustrating a configuration of a server included in an exercise amount information management system according to an exemplary embodiment; and
FIGS. 9 to 11 are flowcharts illustrating methods of managing information related to an exercise amount according to various exemplary embodiments.

Hereinafter, exemplary embodiments will be described in more detail with reference to the accompanying drawings.

In the following description, the same reference numerals are used for the same elements when they are depicted in different drawings. The matters defined in the description, such as detailed construction and elements, are provided to assist in a comprehensive understanding of the exemplary embodiments. Thus, it is apparent that the exemplary embodiments can be carried out without those specifically defined matters. Also, functions or elements known in the related art are not described in detail since they would obscure the exemplary embodiments with unnecessary detail.

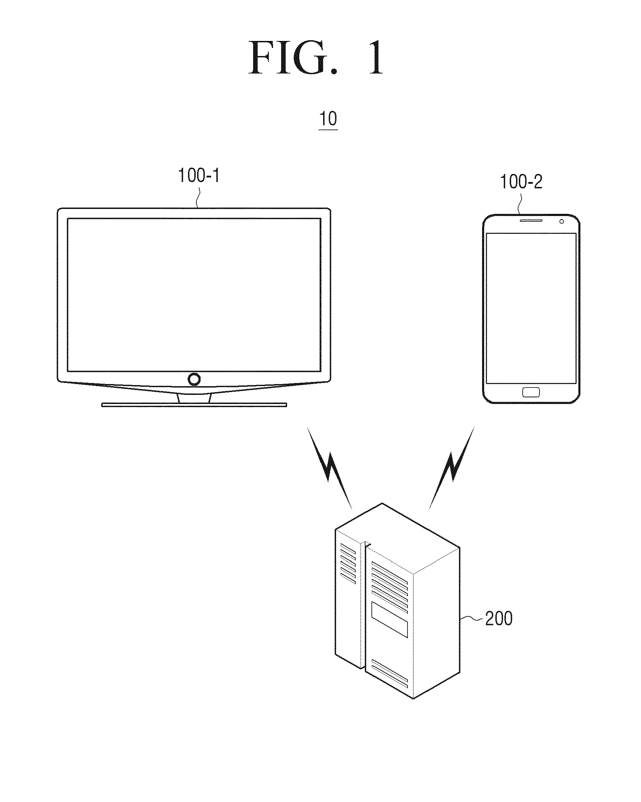

Referring to FIG. 1, an exercise amount information management system 10 according to an exemplary embodiment includes a plurality of display apparatuses 100-1 and 100-2 and a health care server 200. Although a television (TV) 100-1 and a portable phone 100-2 have been illustrated as the display apparatuses 100-1 and 100-2 in FIG. 1, the display apparatuses are merely exemplary. The display apparatuses may be implemented with a personal computer (PC), a laptop PC, a tablet PC, a personal digital assistant (PDA), or the like.

The display apparatuses 100-1 and 100-2 (hereinafter, simply referred to as 100) perform user certification. At this time, the user certification may be performed using at least one from among an identification (ID)/password, face recognition, iris recognition, and fingerprint recognition. The exercise amount information management system may manage exercise amount information through the user certification and the user may receive an exercise amount information management service according to an exemplary embodiment using the plurality of display apparatuses 100-1 and 100-2 without regard to time and a place.

Then, the display apparatus 100 acquires body information of the user and exercise goal information of the user. At this time, the body information of the user may include a height and a weight of the user and the display apparatus 100 may acquire the body information of the user using a digital scale and a depth camera. At this time, the body information of the user including the height and weight of the user is merely illustrative and the body information of the user may further include, for example, a pulse, a blood pressure, a body temperature, and a brainwave.

In addition, the display apparatus 100 acquires exercise goal information of the user according to the setting-up of the user. At this time, the exercise goal information may include at least one from among a goal weight (weight loss goal), amount of calories to be burned, an exercise duration, and exercise content to be used by the user.

Although the user may acquire the body information of the user and the exercise goal information of the user from only one display apparatus 100, this is merely illustrative and the user may acquire the body information of the user and the exercise goal information of the user through the plurality of display apparatuses 100-1 and 100-2.

Then, the display apparatus 100 transmits the acquired body information and exercise goal information of the user to the health care server 200.

The health care server 200 receives and stores the body information and exercise goal information of the user transmitted from the display apparatus 100.

The display apparatus 100 measures exercise amount information using at least one exercise content. Specifically, the display apparatus 100 may measure the exercise amount information using content, programs, and applications which are capable of measuring the exercise amount information of the user. The display apparatuses may measure the exercise amount information using a depth camera, an arm band, a pulsimeter, and a blood pressure gauge. At this time, the exercise amount information may include any one of exercise content information used by the user, exercise time information of the user, and information related to burned calories.

The display apparatus 100 may measure the exercise amount information using metadata stored together when exercise applications or exercise content are installed. For example, when an application A which uses metadata is executed for 10 minutes, if the metadata including information related to calories to be burned of 10 Kcal is received, the display apparatus 100 may measure the exercise amount information using the received metadata. At this time, the metadata may be stored together when the exercise applications or the exercise content are installed, but this is merely illustrative and the metadata may be received in real time from the outside when the exercise application or the exercise content are installed.

The display apparatus 100 may transmit the measured exercise amount information to the health care server 200.

The health care server 200 stores the exercise amount information received through the plurality of display apparatuses 100-1 and 100-2. The health care server 200 integrally calculates the exercise amount information received from the plurality of display apparatuses 100-1 and 100-2 to generate the integrated exercise amount information. At this time, when calorie units of the exercise amount information received from the plurality of display apparatuses 100-1 and 100-2 are different from each other, the health care server 200 may match the calorie units to generate the integrated exercise amount information. For example, when calorie units of exercise amount information received from a display apparatus A is "cal", calorie units of exercise amount information received from a display apparatus B is "Kcal", and calorie units of exercise amount information received from a display apparatus C is "metabolic equivalent of task (METs)", the health care server 200 converts the calorie units of the exercise amount information received from the display apparatuses A, B, and C into units of "cal" and perform integration calculation on the received exercise amount information.

The health care server 200 may divide the integrated exercise amount information related to a display apparatus, dates, and content, and store the divided information.

The health care server 200 may generate health care data for generating a health care UI using the received body information and exercise goal information of the user and the received integrated exercise amount information.

When a transmission request of the health care data is received from any one of the plurality of display apparatuses 100-1 and 100-2, the health care server 200 transmits the health care data to the display apparatus which transmits the transmission request. The health care data may include changed body information of the user, information related to whether or not the user achieves an exercise goal, recommended exercise content, and the like.

Then, the display apparatus 100 generates a health care UI using the received health care data and display the generated health care UI. Here, the health care UI may display the integrated exercise amount information integrally calculated in the health care server 200 for dates or content, using graphs. In addition, the health care UI may display the changed body information of the user and the information related to whether the user achieves the exercise goal.

By the exercise amount information management system 10 described above, the user may intuitively check changes in his/her health state and may integrally maintain his/her health using a plurality of exercise content and at least one display apparatus.

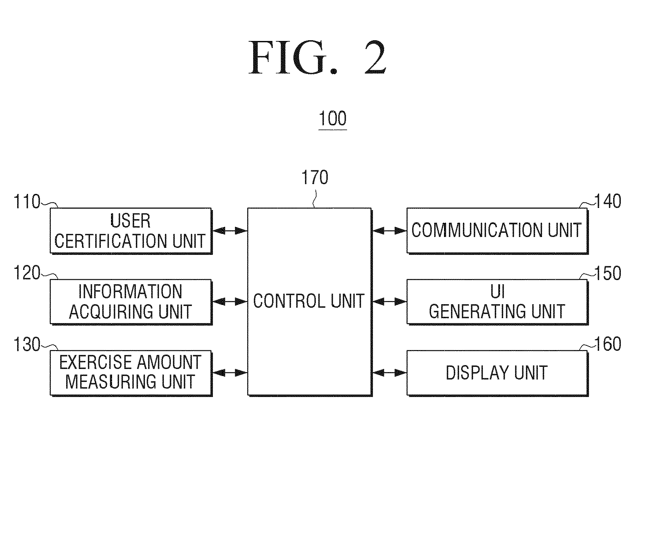

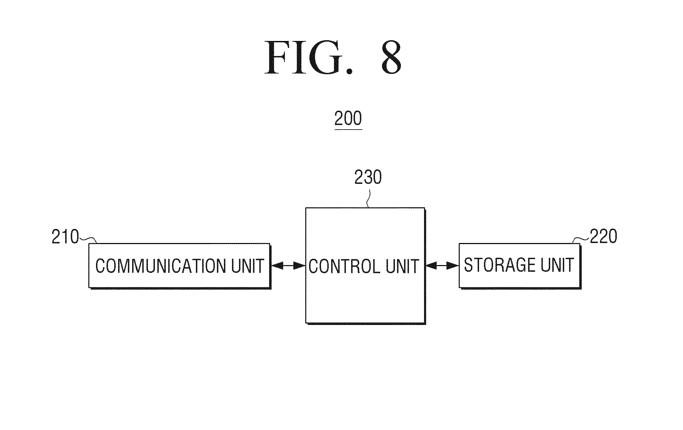

Hereinafter, the display apparatus 100 and the health care server 200 according to an exemplary embodiment will be described with reference to FIGS. 2 and 8.

FIG. 2 is a block diagram illustrating a configuration of the display apparatus according to an exemplary embodiment. As shown in FIG. 2, the display apparatus 100 includes a user certification unit 110, an information acquiring unit 120, an exercise amount measuring unit 130, a communication unit 140, a UI generating unit 150, a display unit 160, and a control unit 170.

The user certification unit 110 performs certification for a user who may receive an exercise amount information management service. The user certification unit 110 may perform the user certification using an ID/password, but this is merely illustrative and the user certification unit 110 may perform the user certification using face recognition, iris recognition, or fingerprint recognition.

The information acquiring unit 120 acquires body information and exercise goal information of the user. Specifically, the information acquiring unit 120 may acquire the body information of the user using an auxiliary device such as a digital scale or a depth camera. In particular, the information acquiring unit 120 may acquire weight information of the user using the digital scale and height information of the user using the depth camera. However, this is merely illustrative and the information acquiring unit 120 may acquire blood pressure information of the user using a blood pressure gauge and pulse information of the user using a pulsimeter.

The information acquiring unit 120 may acquire exercise goal information of the user through setting-up of the user. At this time, the exercise goal information of the user may include at least one selected from among a goal weight of the user (or weight loss goal), an amount of calorie to be burned, an exercise duration, and exercise content to be used by the user.

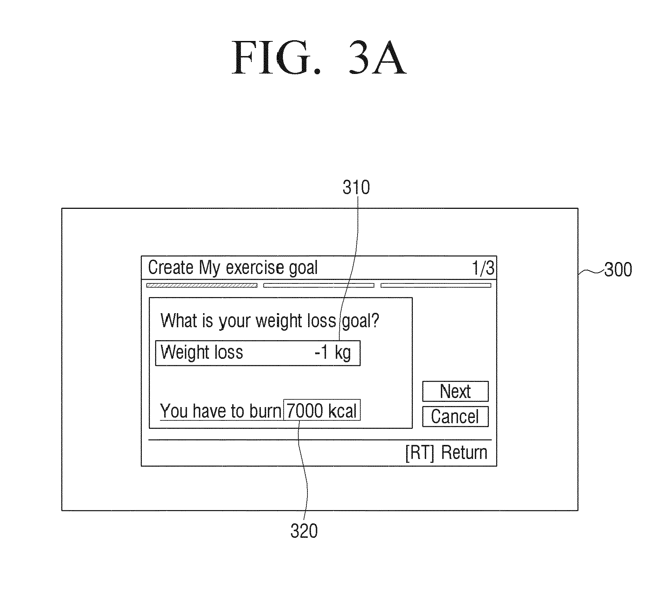

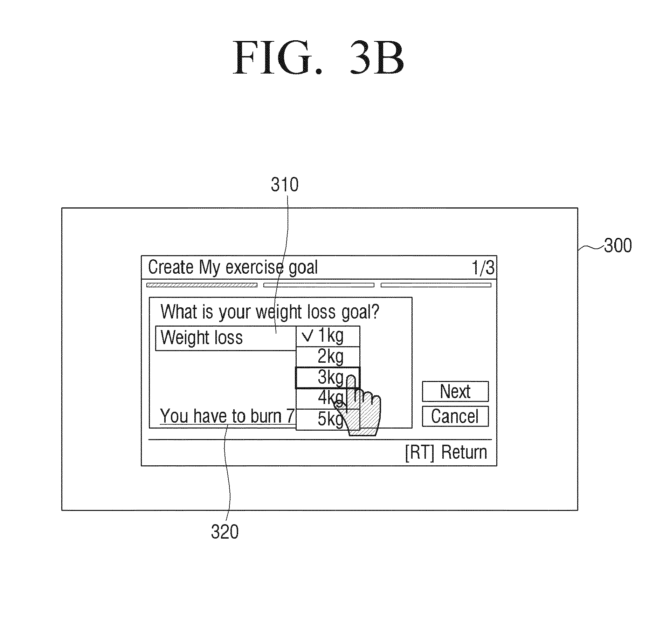

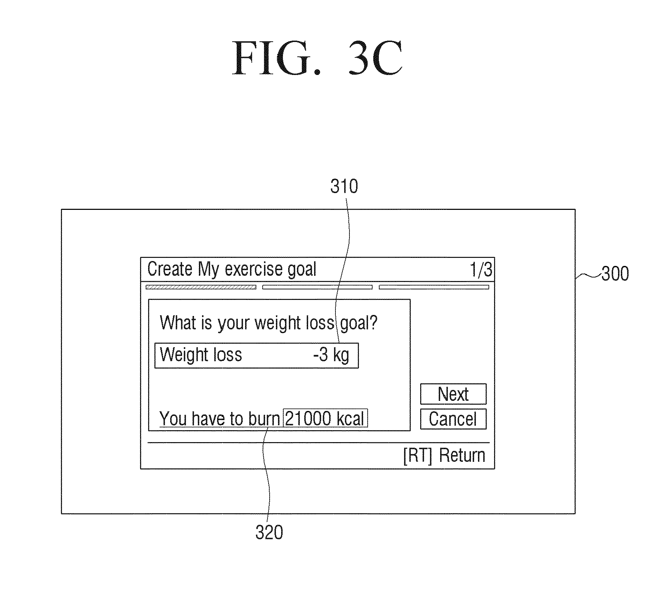

For example, the information acquiring unit 120 may input the weight loss goal of the user using an exercise goal input UI as shown in FIGS. 3A and 3B. As shown in FIG. 3A, when the weight loss goal is 1 kg, the exercise goal input UI 300 may display the weight loss goal and the amount of calories to be burned corresponding to the weight loss goal through the plurality of menus 310 and 320. At this time, when the menu 310 which displays the weight loss goal is selected, as shown in FIG. 3B, the user may change the weight loss goal to a value desired by himself/herself. When the weight loss desired by the user is 3 kg, as shown in FIG. 3C, the user may select 3 kg through the menu 310. When the weight loss of 3 kg is selected, the exercise goal input UI 300 displays changed weight loss goal and an amount of calories to be burned in the plurality of menus 310 and 320.

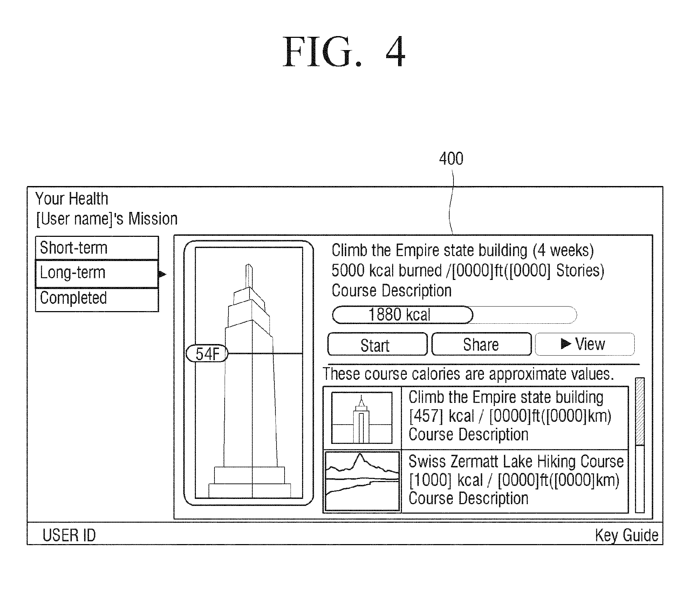

In addition, the information acquiring unit 120 may cause the user to set an exercise goal using a mission list 400 as shown in FIG. 4. At this time, the mission list 400 is a list which guides an amount of calories to be burned to the user by displaying a mission corresponding to the amount of calories to be burned by the user together. For example, as shown in FIG. 4, the mission list 400 may inform the user that climbing the Empire state building would burn 457 kcal. The user may recognize the calories burned by performing the mission through the mission list easily and interestingly.

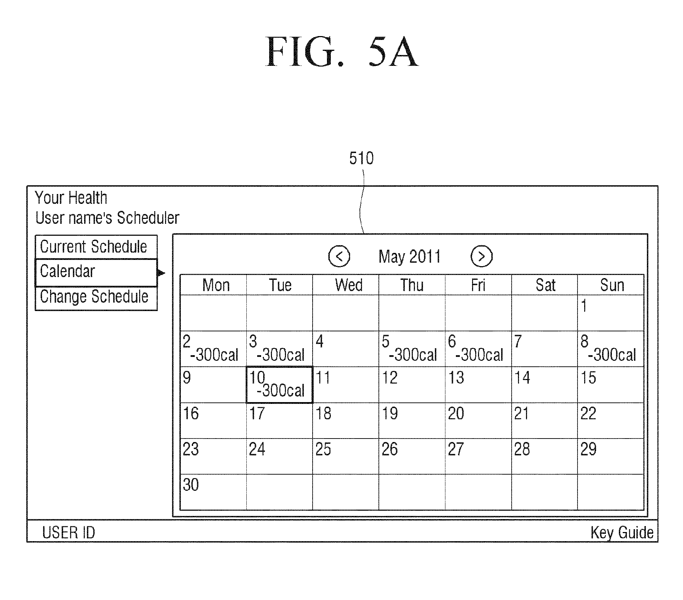

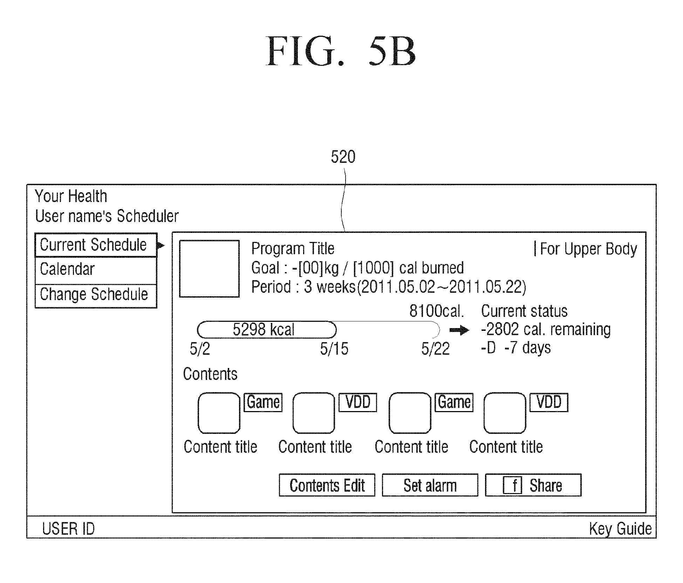

In addition, the information acquiring unit 120 may set an exercise goal amount for dates and exercise content to be used by the user. For example, the information acquiring unit 120 may set an exercise goal amount for dates using a calendar UI 510 as shown in FIG. 5A, or set exercise programs, applications, and exercise content to be used by the user using an exercise content setting UI 520 as shown in FIG. 5B.

The exercise amount measuring unit 130 measures exercise amount information of the user. At this time, the exercise amount information may include any one selected from among exercise content information used by the user, an exercise time information of the user, and information related to burned calories.

In particular, the exercise amount measuring unit 130 may calculate burned calories using a depth camera and an arm band. Specifically, the exercise amount measuring unit 130 captures motion of the user using the depth camera. At this time, the exercise amount information measuring unit may divide a body of the user into a plurality of blocks (for example, a left hand area, a right hand area, a left leg area, a right leg area, a body area, and the like) and determine the motion of the user. The exercise amount measuring unit 120 compares the captured motion of the user with a motion model matched with the preset calories to be burned and stored. Then, the exercise amount measuring unit 130 may calculate similarity of the motion of the user to the motion model to measure the burned calories.

In addition, the exercise amount measuring unit 130 may measure exercise amount information using metadata related to exercise content or exercise applications. At this time, information related to an average amount of burned calories in an hour is stored in the metadata. For example, information, in which an amount of burned calories when an application A is executed, is 1000 cal per hour, is included in metadata related to the application A. When the application A is used for 1.5 hours, the exercise amount measuring unit 130 calculates the amount of calories burned using the application A as 1000 cal x 1.5=1500 cal.

However, the above-described exemplary embodiment is merely illustrative and the amount of burned calories may be measured through various methods. For example, the exercise amount measuring unit 130 may measure an amount of burned calories by the motion of the user using a pulse of the user.

The communication unit 140 performs communication with the external health care server 200. At this time, the communication unit 140 may use a Wi-fi module, Ethernet, and the like, but the inventive concept is not limited thereto.

In particular, the communication unit 140 may transmit the body information, exercise goal information, and exercise amount information of the user output from the information acquiring unit 120 and the exercise mount measuring unit 130 to the health care server 200.

In addition, the communication unit 140 may receive health care data from the health care server 200. Here, the health care data is information generated using the body information, exercise goal information, and integrated exercise amount information of the user in the health care server 200. The health care data may include changed body information of the user, information related to whether the user achieves an exercise goal, and recommended exercise content. However, the health care data is merely illustrative and may not be processed data but may be data which includes the body information, exercise goal information, and integrated exercise amount information of the user.

The UI generating unit 150 may generate various UIs configured to provide an exercise amount information management service. In particular, the UI generation unit 150 may generate the various UIs configured to input the exercise goals as shown in FIGS. 3A to 5B described above.

In addition, the UI generation unit 150 may generate a health care UI using the health care data received through the communication unit 140. The health care UI may display the integrated exercise amount information integrally calculated in the health care server 200 for dates or content using graphs and display the changed body information of the user and the information related to whether or not the user achieves the exercise goal.

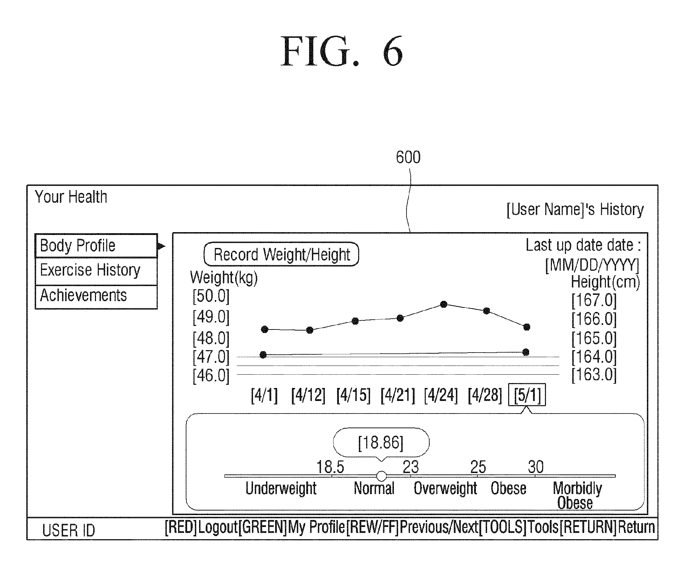

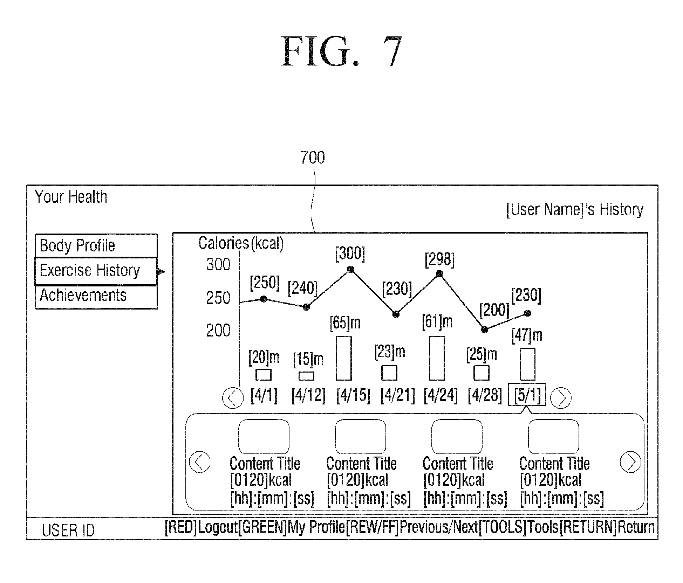

For example, as shown in FIG. 6, a health care UI 600 may display the changed body information of the user for dates through a graph. In addition, as shown in FIG. 7, a health care UI 700 may display an amount of calories burned by the user for dates. The health care UI 700 may include content used by the user and an amount of calories burned when the user uses corresponding content.

The display unit 160 displays the signal-processed image data under control of the control unit 170. In addition, the display unit 160 may display various UIs generated in the UI generating unit 150.

The control unit 170 controls an overall operation of the display apparatus 100 according to an input user command. In addition, the control unit 170 controls the user certification unit 110, the information acquiring unit 120, the exercise amount measuring unit 130, the communication unit 140, the UI generation unit 150, and the display unit 160 described above.

In particular, the control unit 170 may analyze exercise content of the user and body changes for exercise content and provide recommended exercise content to the user. The user may intuitively check his/her health state through the display apparatus 100 according to the exemplary embodiment described above and integrally care for his/her health using the plurality of exercise content.

FIG. 8 is a block diagram illustrating a configuration of the health care server 200 according to an exemplary embodiment. As shown in FIG. 8, the health care server 200 includes a communication unit 210, a storage unit 220, and a control unit 230.

The communication unit 120 performs communication with the plurality of display apparatuses 100-1 and 100-2. In particular, the communication unit 210 may receive body information, exercise goal information, and exercise amount information of the user from the plurality of display apparatuses 100-1 and 100-2. In addition, the communication unit 210 may transmit health care data generated by the control unit 230 to the display apparatus 100.

The storage unit 220 stores the body information, exercise goal information, and exercise amount information of the user received from the plurality of display apparatuses 100-1 and 100-2. At this time, the storage unit 220 may store the pieces of information described above for users.

The control unit 230 controls an overall function and operation of the health care server 200 based on an exercise amount information management service provider. In particular, the control unit 230 may control the communication 210 and the storage unit 220 to care health of the user.

In particular, the control unit 230 integrally calculates the exercise amount information of the user received from the plurality of display apparatuses 100-1 and 100-2 for users, and generates the integrated exercise amount information. Specifically, the control unit 230 integrally calculates only exercise amount information of a user A among the exercise amount information received from the plurality of display apparatus 100-1 and 100-2 and generates integrated exercise amount information for the user "A".

For example, when the user A burned 300 cal using exercise content a on December 1, 2011, burned 100 cal using the exercise content a on December 3, 2011, burned 500 cal using exercise content b on December 2, 2011, burned 200 cal using exercise content c on December 1, 2011, and burned 600 cal using the exercise content c on December 2, 2011, the control unit 230 may generate the integrated exercise amount information as Table 1.

[Table]

|  | Exercise content a | Exercise content b | Exercise content c | Total |
|---|---|---|---|---|
| December 1, 2011 | 300cal | - | 200cal | 500cal |
| December 2, 2011 | - | 500cal | 600cal | 1100cal |
| December 3, 2011 | 100cal | - | - | 100cal |

Therefore, using the integrated exercise amount information as described above, the user may check total exercise amount information, exercise amount information related to content, and exercise amount for dates used from December 1, 2011 to December 3, 2011.

At this time, the integrated exercise amount information for the user A may include exercise content used through the plurality of display apparatuses 100-1 and 100-2, burned calories, exercise date information as well as additional exercise amount information such as a content using time, and a burned amount of calories accumulated for dates.

When calorie units of the exercise amount information received from the plurality of display apparatuses 100-1 and 100-2 are different from each other, the control unit 230 may match the calorie units and generate the integrated exercise amount information. For example, when the calorie unit of the exercise amount information received from the display A is "cal", the calorie unit of the exercise amount information received from the display B is "Kcal", the calorie unit of the exercise amount information received from the display C is "METs", the health care server 200 may convert the exercise amount information transmitted from the display apparatuses A, B, and C into the exercise amount information in unit of "cal" and perform an integration calculation. Here, the METs unit may be converted into cal unit by the following equation.

[Equation 1]

$$\text{Final exercise amount (cal)} = 1.20(\text{METs}-1)*T*W*A/Wm$$

Here, METs is a value of the exercise amount, T is a exercise time (min), W is a weight (kg), A is an age information coefficient, and Wm is a sex information coefficient (man: 63 and woman:52).

The age information coefficient may be defined as the following Table 2.

[Table 2]

| Age | Man | Woman |
|---|---|---|
| 20~ | 1.0 | 1.0 |
| 30~ | 0.95 | 0.95 |
| 40~ | 0.93 | 0.91 |
| 50~ | 0.95 | 0.90 |
| 60~ | 0.91 | 0.90 |
| 70~ | 0.89 | 0.89 |

In addition, the control unit 230 may generate health care data using the body information, exercise goal information, and integrated exercise amount information of the user stored in the storage unit 220. At this time, the health care data may include changed body information of the user, information on whether the user achieves the exercise goal, recommended exercise content, and the like. However, the health care data is merely illustrative and may not be processed data but may be data which includes the body information, exercise goal information, and integrated exercise amount information of the user.

Through the health care server 200 as described above, the user may monitor his/her health using the plurality of display apparatuses 100-1 and 100-2 without regard to time and a place.

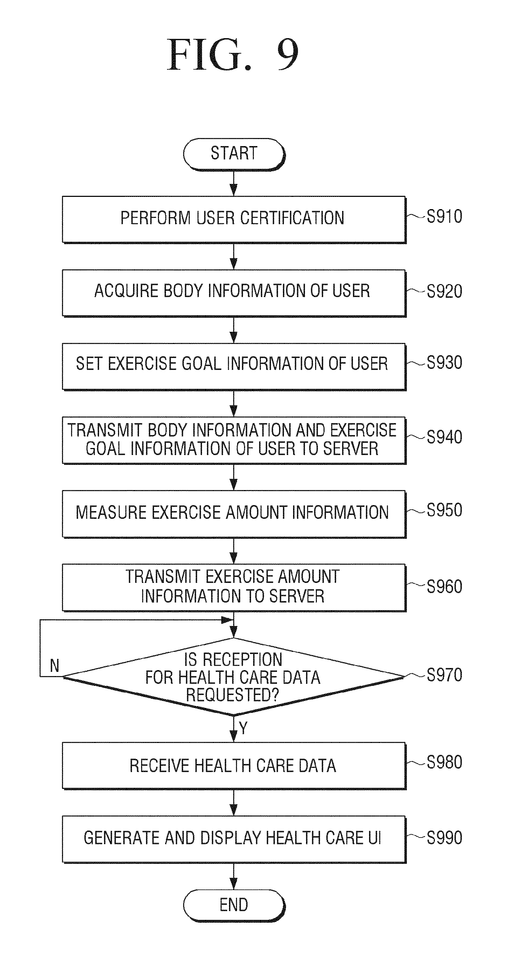

Hereinafter, health care methods according to various exemplary embodiments will be described with reference to FIGS. 9 to 11.

FIG. 9 is a flowchart illustrating a health care method of the display apparatus according to an exemplary embodiment.

First, the display apparatus 100 performs user certification (S910). At this time, the display apparatus 100 may perform certification using an ID/password, face recognition, iris recognition, and fingerprint recognition.

The display apparatus 100 acquires body information of the user (S920). At this time, the body information of the user may include at least one from among a height and a weight of the user. The display apparatus 100 may acquire height information of the user using a depth camera and acquire weight information of the user using a digital scale.

Then, the display apparatus 100 sets exercise goal information of the user (S930). At this time, the exercise goal information of the user may include at least one selected from among a goal weight of the user, an amount of calories to be burned, an exercise duration, and exercise content to be used by the user. In particular, the display apparatus 100 may input the exercise goal information through various exercise goal input UIs as shown in FIGS. 5A to 5B.

The display apparatus 100 transmits the acquired body information and exercise goal information to the health care server 200 (S940).

Then, the display apparatus 100 measures exercise amount information (S950). At this time, the exercise amount information may include at least one selected from the among exercise content information used by the user, exercise time information of the user, and burned calorie information calculated using a depth camera and an arm band.

The display apparatus 100 transmits the calculated exercise amount information to the health care server 200 (S960).

Then, the display apparatus 100 determines whether or not reception of health care data is requested from the user (S970).

When it is determined that the reception for health care data is requested (S970-Y), the display apparatus 100 receives the health care data from the health care server 200 (S980). At this time, the health care data may include changed body information of the user, information on whether the user achieves the exercise goal, or recommended exercise content. However, the health care data described above is merely illustrative and may not be processed data but may be data which includes the body information, exercise goal information, and integrated exercise amount information of the user.

Then, the display apparatus 100 generates a health care UI and displays the generated health care UI (S990). At this time, the health care UI may display the integrated exercise amount information integrally calculated in the health care server 200 for dates or content, using graphs and display the changed body information of the user and the information on whether the user achieves the exercise goal. For example, the health care UI may be the UIs as shown in FIGS. 6 and 7.

By the display apparatus 100 described above, the user may integrally receive an exercise amount information management service using a plurality of exercise content. In addition, the user may intuitively check on his/her health state.

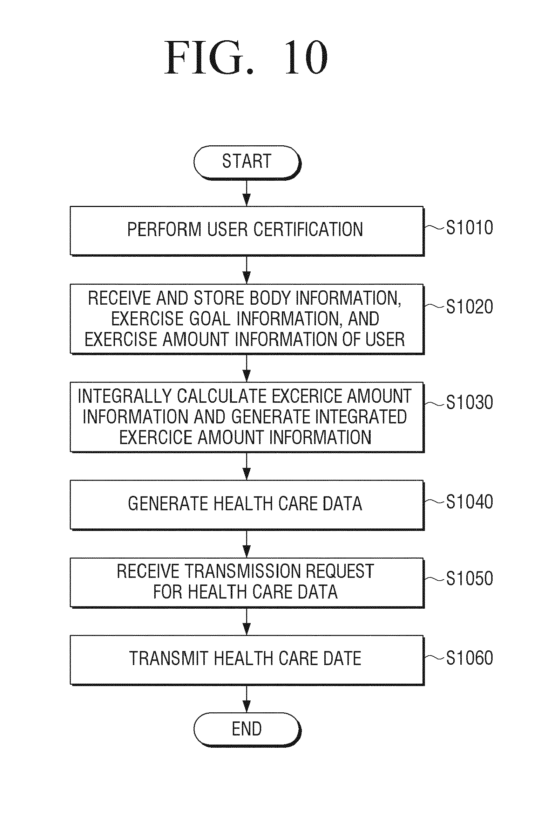

FIG. 10 is a flowchart illustrating a health care method of the health care server 200 according to an exemplary embodiment.

First, the health management server 200 performs user certification (S1010). At this time, the user is a user who is certified from the display apparatus 100 and receives an exercise amount information management service.

Then, the health care server 200 receives and stores the body information, exercise goal information, and exercise amount information of the user from the display apparatus 100 (S1020). At this time, the body information, exercise goal information, and exercise amount information of the user may be received from the plurality of display apparatuses 100-1 and 100-2.

The health care server 200 integrally calculates the received exercise amount information and generates integrated exercise amount information (S1030). At this time, the integrated exercise amount information is information in which the exercise amount information generated using the plurality of exercise content and the plurality of display apparatuses are integrated and managed for users.

Then, the health care server generates health care date (S1040). At this time, the health care data is data generated using the body information, exercise goal information, and integrated exercise amount information of the user. In particular, the health care data may include changed body information of the user, information on whether or not a user achieves the exercise goal, recommended exercise content, and the like, but the health care data is merely illustrative and may not be processed data but may be data which includes the body information, exercise goal information, and exercise amount information of the user.

Then, the health care server 200 receives a transmission request for the health care data from the display apparatus 100 (S1050).

When the transmission request for the health care data is received, the health care server 200 transmits the health care data to the external display apparatus 100 (S1060).

Through the health care server 200 as described above, the user may maintain his/her heath using the plurality of display apparatuses 100 without regard to a time and a place.

On the other hand, the above-described exemplary embodiment has described that the health care data is generated in advance before the transmission request of the health care data, but this is merely illustrative and the health care server 200 may generate the health care data after the transmission request for the health care data is received.

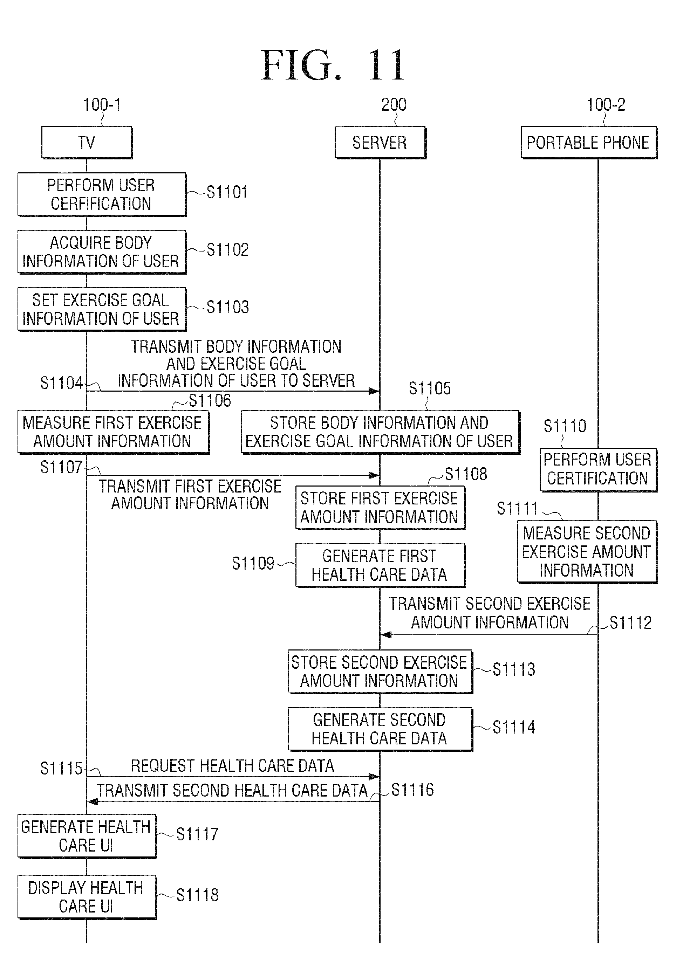

FIG. 11 is a sequence illustrating a health care method of the exercise amount information management system according to another exemplary embodiment.

First, the TV 100-1 performs user certification (S1101). Then, the TV 100-1 acquires body information of the user (S1102) and sets exercise goal information of the user (S1103).

Then, the TV 100-1 transmits the acquired body information of the user and the set exercise goal information of the user to the heath care server 200 (S1104).

The server 200 stores the received body information and exercise goal information of the user (S1105).

Then, the TV 100-1 measures first exercise amount information (S1106), and transmits the measured first exercise amount information to the external health care server 200 (S1107).

The health care server 200 stores the received first exercise amount information (S1108). Then, the health care server 200 generates first health care data using the received body information and exercise goal information of the user and the received first exercise amount information (S1109).

Then, the portable phone 100-2 performs user certification for the same user as the user that was certified via the TV 100-1 (S1110).

The portable phone 100-2 measures second exercise amount information (S1111), and transmits the measured second exercise amount information to the external health care server 200 (S1112).

When the second exercise amount information is received, the health care server 200 stores the second exercise amount information (S1113). Then, the health care server 200 generates second health care data using the received body information and exercise goal information of the user, the received first exercise amount information, and the integrated exercise amount information in which the first exercise amount information and the second exercise amount information are integrally calculated.

Then, the TV 100-1 requests health care data from the health care server 200 (S1115).

When a request signal for health care data is received, the health care server 200 transmits the latest second health care data to the TV 100-1 (S1116).

When the second health care data is received, the TV 100-1 generates a health care UI using the second health care data (S1117), and displays the generated health care UI (S1118).

Using the exercise amount information management system 10 described above, the user may intuitively check his/her health state and integrally maintain his/her health using the plurality of display apparatuses 100-1 and 100-2.

In addition, a program for executing the health care methods according to the above-described various exemplary embodiments may be recorded in various types of recording media. Specifically, a program code for executing health care methods may be stored in various types of recording media readable by a terminal such as a random access memory (RAM), a flash memory, a read only memory (ROM), an erasable programmable ROM (EPROM), an electronically erasable and programmable ROM (EEPROM), a register, a hard disc, a removable disc, a memory card, a USB memory, a compact disc ROM (CD-ROM).

The foregoing embodiments are merely exemplary and are not to be construed as limiting the present inventive concept. The exemplary embodiments can be readily applied to other types of apparatuses. Also, the description of the exemplary embodiments is intended to be illustrative, and not to limit the scope of the claims, and many alternatives, modifications, and variations will be apparent to those skilled in the art.

**Claims**

1. A method of managing information related to an exercise amount in a display apparatus, the method comprising:

    measuring exercise amount information of at least one user using a plurality of exercise content and transmitting the measured exercise amount information to a server; and
    receiving exercise amount information which has been integrally calculated, from the server and displaying the received exercise amount information on the display apparatus.

2. The method as claimed in claim 1, wherein the exercise amount information includes at least one from among exercise content information used by the user, exercise time information of the user, and information for burned calories calculated using at least one from among a depth camera and an arm band.

3. The method as claimed in claim 1 or 2, further comprising acquiring body information of the user and exercise goal information of the user and transmitting the acquired information to the server.

4. The method as claimed in claim 3, wherein the body information of the user includes at least one from among a height and a weight of the user,
wherein the height of the user is acquired using the depth camera and the weight of the user is acquired by a digital scale, and
wherein the exercise goal information of the user includes at least one from among a goal weight of the user, an amount of calories to be burned, an exercise duration, and exercise content to be used by the user.

5. The method as claimed in claim 3 or 4, further comprising receiving the body information of the user and the exercise goal information of the user from the server,
wherein the displaying includes:

generating a health care user interface (UI) using at least one from among the body information of the user, the exercise goal information of the user, and the exercise amount information of the user: and
displaying the generated health care UI.

6. The method as claimed in claim 5, wherein the health care UI displays the exercise amount information measured using the plurality of content, using a graph related to at least one from among dates and content.

7. The method as claimed in claim 5 or 6, wherein the health care UI displays changed body information of the user and information related to whether the user achieves an exercise goal.

8. The method as claimed in any one of claims 3 to 7, further comprising display an exercise goal input UI configured to input the exercise goal information of the user,
wherein the exercise goal input UI includes a mission list configured to guide an amount of calories to be burned when a specific mission is performed.

9. The method as claimed in any one of claims 3 to 8, further comprising analyzing the body information of the user and the exercise amount information of the user and providing recommendation exercise contents for the user.

10. A display apparatus, comprising:

an exercise amount measuring unit configured to measure exercise amount information of at least one user using a plurality of exercise content;
a communication unit configured to transmit the measured exercise amount information to a server; and
a display unit configured to receive exercise amount information which has been integrally calculated from the server and display the received exercise amount information.

11. The display apparatus as claimed in claim 10, wherein the exercise amount information includes at least one from among exercise content information used by the user, exercise time information of the user, and information for burned calories calculated using at least one from among a depth camera and an arm band.

12. The display apparatus as claimed in claim 10 or 11, further comprising an information acquiring unit configured to acquire body information of the user and exercise goal information of the user,
wherein the communication unit is arranged to transmit the acquired body information of the user and the acquired exercise goal information of the user to the server.

13. The display apparatus as claimed in claim 12, wherein the body information of the user includes at least one from among a height and a weight of the user,
wherein the height of the user is acquired using the depth camera and the weight of the user is acquired by a digital scale, and
wherein the exercise goal information of the user includes at least one from among a goal weight of the user, an amount of calories to be burned, an exercise duration, and exercise content to be used by the user.

14. The display apparatus as claimed in claim 12 or 13, further comprising a user interface (UI) generating unit configured to generate a health care UI using the body information of the user, the exercise goal information of the user, and the exercise amount information of the user received through the communication unit,
wherein the display unit is arranged to display the generated health care UI.

15. The display apparatus as claimed in claim 14, wherein the health care UI displays the exercise amount information measured using the plurality of content, using a graph related to at least one from among dates and content.

# FIG. 1

10

100-1

100-2

200

# FIG. 2

100

170

110 — USER CERTIFICATION UNIT

120 — INFORMATION ACQUIRING UNIT

130 — EXERCISE AMOUNT MEASURING UNIT

CONTROL UNIT

140 — COMMUNICATION UNIT

150 — UI GENERATING UNIT

160 — DISPLAY UNIT

# FIG. 3A

310

Create My exercise goal | 1/3

300

What is your weight loss goal?

Weight loss | -1 kg

You have to burn | 7000 kcal

Next

Cancel

[RT] Return

320

# FIG. 3B

310

300

Create My exercise goal                    1/3

What is your weight loss goal?

| Weight loss | ✓ 1kg |
|             | 2kg |
|             | 3kg |
|             | 4kg |
| You have to burn 7 | 5kg |

Next

Cancel

[RT] Return

320

# FIG. 3C

310

300

Create My exercise goal                    1/3

What is your weight loss goal?

Weight loss              -3 kg

Next
Cancel

You have to burn 21000 kcal

[RT] Return

320

# FIG. 4

400

Your Health
[User name]'s Mission

| Short-term |
| Long-term |
| Completed |

54F

Climb the Empire state building (4 weeks)
5000 kcal burned /[0000]ft([0000] Stories)
Course Description

1880 kcal

| Start | Share | ▶ View |

These course calories are approximate values.

Climb the Empire state building
[457] kcal / [0000]ft([0000]km)
Course Description

Swiss Zermatt Lake Hiking Course
[1000] kcal / [0000]ft([0000]km)
Course Description

USER ID

Key Guide

17

# FIG. 5A

510

| Your Health<br>User name's Scheduler | | | | | | | |
|---|---|---|---|---|---|---|---|

| Current Schedule |
|---|
| Calendar |
| Change Schedule |

| ⊘ May 2011 ⊙ | | | | | | |
|---|---|---|---|---|---|---|
| Mon | Tue | Wed | Thu | Fri | Sat | Sun |
| | | | | | | 1 |
| 2 -300cal | 3 -300cal | 4 | 5 -300cal | 6 -300cal | 7 | 8 -300cal |
| 9 | 10 -300cal | 11 | 12 | 13 | 14 | 15 |
| 16 | 17 | 18 | 19 | 20 | 21 | 22 |
| 23 | 24 | 25 | 26 | 27 | 28 | 29 |
| 30 | | | | | | |

| USER ID | Key Guide |
|---|---|

# FIG. 5B

520

Your Health
User name's Scheduler

| Current Schedule |
| Calendar |
| Change Schedule |

Program Title                                    | For Upper Body
Goal : -[00]kg / [1000] cal burned
Period : 3 weeks(2011.05.02~2011.05.22)

8100cal.   Current status
( 5298 kcal )          ➡ -2802 cal. remaining
5/2            5/15         5/22    -D -7 days

Contents

[Game]    [VDD]    [Game]    [VDD]

Content title   Content title   Content title   Content title

| Contents Edit |   | Set alarm |   | [f] Share |

USER ID                                              Key Guide

# FIG. 6

600

| Your Health | | [User Name]'s History |

Body Profile ▶
Exercise History
Achievements

Record Weight/Height

Weight(kg)
[50.0]
[49.0]
[48.0]
[47.0]
[46.0]

[4/1]  [4/12]  [4/15]  [4/21]  [4/24]  [4/28]  [5/1]

Last up date date :
[MM/DD/YYYY]
Height(cm)
[167.0]
[166.0]
[165.0]
[164.0]
[163.0]

[18.86]

18.5        23       25      30

Underweight    Normal    Overweight   Obese   Morbidly
                                              Obese

USER ID | [RED]Logout[GREEN]My Profile[REW/FF]Previous/Next[TOOLS]Tools[RETURN]Return

# FIG. 7

700

| Your Health | [User Name]'s History |

Body Profile
Exercise History
Achievements

Calories (kcal)
300
250
200

[250] [240] [300] [230] [298] [200] [230]

[20]m [15]m [65]m [23]m [61]m [25]m [47]m

[4/1] [4/12] [4/15] [4/21] [4/24] [4/28] [5/1]

Content Title
[0120]kcal
[hh]:[mm]:[ss]

Content Title
[0120]kcal
[hh]:[mm]:[ss]

Content Title
[0120]kcal
[hh]:[mm]:[ss]

Content Title
[0120]kcal
[hh]:[mm]:[ss]

USER ID    [RED]Logout[GREEN]My Profile[REW/FF]Previous/Next[TOOLS]Tools[RETURN]Return

# FIG. 8

200

230

210

COMMUNICATION UNIT ◄──► CONTROL UNIT ◄──► STORAGE UNIT

220

# FIG. 9

```
            ┌─────────────┐
            │    START     │
            └──────┬──────┘
                   ▼
   ┌───────────────────────────────┐
   │  PERFORM USER CERTIFICATION    │──S910
   └───────────────┬───────────────┘
                   ▼
   ┌───────────────────────────────┐
   │  ACQUIRE BODY INFORMATION OF USER │──S920
   └───────────────┬───────────────┘
                   ▼
   ┌───────────────────────────────┐
   │ SET EXERCISE GOAL INFORMATION OF USER │──S930
   └───────────────┬───────────────┘
                   ▼
   ┌───────────────────────────────┐
   │ TRANSMIT BODY INFORMATION AND EXERCISE │──S940
   │ GOAL INFORMATION OF USER TO SERVER │
   └───────────────┬───────────────┘
                   ▼
   ┌───────────────────────────────┐
   │ MEASURE EXERCISE AMOUNT INFORMATION │──S950
   └───────────────┬───────────────┘
                   ▼
   ┌───────────────────────────────┐
   │    TRANSMIT EXERCISE AMOUNT    │──S960
   │    INFORMATION TO SERVER       │
   └───────────────┬───────────────┘
                   ▼
           IS RECEPTION
      N   FOR HEALTH CARE DATA      ──S970
           REQUESTED?
                   │Y
                   ▼
   ┌───────────────────────────────┐
   │    RECEIVE HEALTH CARE DATA    │──S980
   └───────────────┬───────────────┘
                   ▼
   ┌───────────────────────────────┐
   │ GENERATE AND DISPLAY HEALTH CARE UI │──S990
   └───────────────┬───────────────┘
                   ▼
            ┌─────────────┐
            │     END      │
            └─────────────┘
```

# FIG. 10

START

PERFORM USER CERTIFICATION — S1010

RECEIVE AND STORE BODY INFORMATION, EXERCISE GOAL INFORMATION, AND EXERCISE AMOUNT INFORMATION OF USER — S1020

INTEGRALLY CALCULATE EXCERICE AMOUNT INFORMATION AND GENERATE INTEGRATED EXCERICE AMOUNT INFORMATION — S1030

GENERATE HEALTH CARE DATA — S1040

RECEIVE TRANSMISSION REQUEST FOR HEALTH CARE DATA — S1050

TRANSMIT HEALTH CARE DATE — S1060

END

# FIG. 11

100-1      200      100-2

| TV | | SERVER | | PORTABLE PHONE |

PERFORM USER CERTIFICATION  ~S1101

ACQUIRE BODY INFORMATION OF USER  ~S1102

SET EXERCISE GOAL INFORMATION OF USER  ~S1103

TRANSMIT BODY INFORMATION AND EXERCISE GOAL
S1104~ INFORMATION OF USER TO SERVER     S1105

~S1106

MEASURE FIRST EXERCISE AMOUNT INFORMATION

STORE BODY INFORMATION AND EXERCISE GOAL INFORMATION OF USER

S1110

PERFORM USER CERTIFICATION

S1107~

TRANSMIT FIRST EXERCISE AMOUNT INFORMATION     ~S1108

STORE FIRST EXERCISE AMOUNT INFORMATION

S1111~

MEASURE SECOND EXERCISE AMOUNT INFORMATION

S1109~ GENERATE FIRST HEALTH CARE DATA

TRANSMIT SECOND EXERCISE AMOUNT INFORMATION  ~S1112

STORE SECOND EXERCISE AMOUNT INFORMATION  ~S1113

GENERATE SECOND HEALTH CARE DATA  ~S1114

S1115~   REQUEST HEALTH CARE DATA

TRANSMIT SECOND HEALTH CARE DATA ~S1116

GENERATE HEALTH CARE UI  ~S1117

DISPLAY HEALTH CARE UI  ~S1118